# EUROPEAN PATENT SPECIFICATION

(11) **EP 3 436 598 B1**
(45) Date of publication and mention of the grant of the patent: **08.01.2025**
(21) Application number: 17717098.2
(22) Date of filing: 31.03.2017
(51) Int. Cl.: C12Q 1/48, G01N 33/574

(54) **METHOD FOR PREDICTING THE RESPONSE OF MELANOMA PATIENTS TO A MEDICAMENT**
VERFAHREN ZUR VORHERSAGE DES ANSPRECHENS EINES MELANOMPATIENTEN AUF EIN MEDIKAMENT
PROCÉDÉ PERMETTANT DE PRÉDIRE LA RÉPONSE DE PATIENTS ATTEINTS DE MÉLANOME À UN MÉDICAMENT

(30) Priority: 31.03.2016 WO PCT/EP2016/163336
(43) Date of publication of application: 06.02.2019
(60) Divisional application: 20184852.0
(73) Proprietor: PamGene B.V., 5211 HH 's-Hertogenbosch (NL); Leiden University Medical Center, 2333 ZA Leiden (NL)
(72) Inventor: RUIJTENBEEK, Robby, 3515 CW Utrecht (NL); HOVESTAD-BIJL, Liesbeth Coosje, 5231 PK 's-Hertogenbosch (NL); DE WIJN, Richard, 6542 PS Nijmegen (NL); VAN DER BURG, Sjoerd H., 2742 BN Waddinxveen (NL); VERDEGAAL, Elizabeth M. E., 2171 BL Sassenheim (NL)
(74) Representative: De Clercq & Partners
(86) International application number: PCT/EP2017/057643
(87) International publication number: WO 2017/167942

(56) References cited:
- EP-A1- 2 309 271
- WO-A1-2012/131065
- WO-A1-2016/184999
- US-A1- 2005 187 147
- W MAAT ET AL: "Episodic Src activation in uveal melanoma revealed by kinase activity profiling", BRITISH JOURNAL OF CANCER, vol. 101, no. 2, 30 June 2009 (2009-06-30), GB, pages 312 - 319, XP055297914, ISSN: 0007-0920, DOI: 10.1038/sj.bjc.6605172
- W MAAT ET AL: "Application Note -Protein Tyrosine Kinases; Target Discovery", 1 October 2010 (2010-10-01), XP055229565, Retrieved from the Internet <URL:https://www.pamgene.com/upload/image/Applications notes/201009 (10-2010).pdf> [retrieved on 20151119]
- SIGURD FOLKVORD ET AL: "Prediction of Response to Preoperative Chemoradiotherapy in Rectal Cancer by Multiplex Kinase Activity Profiling", INTERNATIONAL JOURNAL OF RADIATION: ONCOLOGY BIOLOGY PHYSICS, PERGAMON PRESS, USA, vol. 78, no. 2, 14 April 2010 (2010-04-14), pages 555 - 562, XP028141113, ISSN: 0360-3016, [retrieved on 20100507], DOI: 10.1016/J.IJROBP.2010.04.036
- ROB RUIJTENBEEK: "kinase activity profiling in tumour tissues by PamChip peptide micorarrays", INTERNET CITATION, 11 December 2007 (2007-12-11), XP002538369, Retrieved from the Internet <URL:http://retina.med.harvard.edu/LITT/Pamgene_12_11_2007.pdf> [retrieved on 20090721]

## Description

### FIELD OF THE INVENTION

The present invention relates to a methods and devices for determining or predicting the response of a patient diagnosed with melanoma to an immunotherapeutic antibody directed against CTLA-4. More specifically, the present invention provides methods which measure phosphatase activity by studying phosphorylation levels and profiles in samples obtained from blood samples of said patients.

### BACKGROUND OF THE INVENTION

Melanoma is a cancer that begins in the melanocytes. Melanocytes, the cells that can become melanoma, are also found in the epidermis. These skin cells make a brown pigment called melanin, which gives the skin its tan or brown colour. Melanin protects the deeper layers of the skin from some of the harmful effects of the sun. For most people, when skin is exposed to the sun, melanocytes make more of the pigment, causing the skin to tan or darken. Melanoma accounts for less than 2% of skin cancer cases but causes a large majority of skin cancer deaths. Melanoma is a cancer that begins in the melanocytes.

Melanoma remains a highly morbid disease and its incidence has continued to rise sharply over the past few decades. Overall incidence rates for melanoma are increasing among men and women. Since 1981, the rate of increase has been about 3% per year. According to estimates from the American Cancer Society, there were about 59,580 new cases of melanoma in the U.S. in 2005, and about 7,700 people died of this disease. Prior to 2009 the only FDA approved treatments for metastatic melanoma included dacarbazine, interferon-alpha, and interleukin-2. However, the prognosis of patients with disseminated disease remained poor, with a 5-year survival rate of 16% or less in the U.S. New treatments such as anti-angiogenic agents, Raf, Mek and other kinase inhibitors and vaccines are currently being developed and may offer improvements in survival for patients with this disease. In addition, the toll of melanoma in terms of "life-years lost" is the highest of all solid tumours in the United States.

For the diagnosis of melanoma a surface skin biopsy, punch biopsy or excisional biopsy is taken. Based on a primary biopsy diagnosis nearby lymph nodes may be biopsied to see if the cancer has spread. Staging of melanoma is based on the American Joint Committee on Cancer (AJCC) TNM system. The T stands for tumour (how far it has grown within the skin and other factors). The T category is assigned a number (from 0 to 4) based on the tumour's thickness (how far down it has grown). N stands for spread to nearby lymph nodes (bean-sized collections of immune system cells, to which cancers often spread first). The N category is assigned a number (from 0 to 3) based on whether the melanoma cells have spread to lymph nodes or are found in the lymphatic channels connecting the lymph nodes. The M category is based on whether the melanoma has metastasized (spread) to distant organs, which organs it has reached. According to these standards the different stages and survival in the US are as follows:

| | |
|---|---|
| • Stage IA : | The 5-year survival rate is around 97%. The 10-year survival is around 95%. |
| • Stage IB : | The 5-year survival rate is around 92%. The 10-year survival is around 86%. |
| • Stage IIA : | The 5-year survival rate is around 81%. The 10-year survival is around 67%. |
| • Stage IIB : | The 5-year survival rate is around 70%. The 10-year survival is around 57%. |
| • Stage IIC : | The 5-year survival rate is around 53%. The 10-year survival is around 40%. |
| • Stage IIIA : | The 5-year survival rate is around 78%. The 10-year survival is around 68%. |
| • Stage IIIB : | The 5-year survival rate is around 59%. The 10-year survival is around 43%. |
| • Stage IIIC : | The 5-year survival rate is around 40%. The 10-year survival is around 24%. |
| • Stage IV : | The 5-year survival rate is about 15%-20%. The 10-year survival is about 10%-15%. |

Melanoma treatment options are based on the stage of the disease and may include: surgery, chemotherapy, targeted therapy, immunotherapy and radiation therapy. Early-stage melanomas can often be cured with surgery alone, but more advanced melanomas can be much harder to treat because standard cancer treatments such as chemotherapy are not very effective. But in recent years, newer types of immunotherapy and targeted therapies have changed the treatment of this disease, and many new treatments have shown a great deal of promise in treating advanced melanomas.

Current immunotherapy approaches for melanoma fall into six main categories: checkpoint inhibitors, oncolytic virus therapies, cancer vaccines, adoptive T cell therapy, monoclonal antibodies, and cytokines. Immune checkpoint inhibitors have been successfully used to treat melanoma. This therapy is based upon the fact that T lymphocytes are critical to antitumor immunity, and this antitumor immunity requires activation by an antigen-specific T cell receptor in the context of costimulatory activation. Excess immune activation is being prevented by a naturally occurring feedback mechanism that leads to the expression of negative costimulatory molecules ("checkpoints"). Examples of such checkpoints are cytotoxic T-lymphocyte antigen 4 (CTLA-4), programmed death 1 (PD-1), T cell immunoglobulin 3, and lymphocyte-activation gene 3. Antibodies directed against these checkpoints may restore or augment an antitumor immune response and produce tumor responses in patients with advanced melanoma. Examples of such antibodies are Ipilimumab (CTLA-4 inhibition), Nivolumab (PD-1 inhibition) and Pembrolizumab (PD-L1 inhibition).

In particular, stage IV melanomas are very hard to cure, as they have already spread to distant lymph nodes or other areas of the body. While the skin tumours can often be removed by surgery or treated with radiation therapy, metastases in internal organs which cannot be removed may be treated with radiation, immunotherapy, targeted therapy, or chemotherapy. Checkpoint inhibitors can be used alone or in combination. Though, not all patients respond to these therapies. Approximately 30% of the patients treated with a checkpoint inhibitor respond to this drug. For combinatorial treatment with checkpoint blockers, approximately 60% of the patients show a positive response.

Unfortunately, most anti-tumour treatments are associated with undesirable side effects, such as profound nausea, vomiting, or severe fatigue. Also, while anti-tumour treatments have been successful, they do not produce significant clinical responses in all patients who receive them resulting in undesirable side effects, delays, and costs associated with ineffective treatment. Therefore, biomarkers that can be used to predict the response of a subject to an antitumor agent prior to administration thereof are greatly needed.

Given the high incidence of melanoma and limited efficacy of current treatments, a melanoma biomarker and assay for a melanoma biomarker is needed.

Also, assays for melanoma biomarkers as an accurate early indicator for therapeutic response typically require taking a surface skin biopsy, punch biopsy or excisional biopsy which are considered unpleasant for the patient and can result in the formation of scar tissue.

In view of the above, there remains a pressing need for improved methods that provide a fast and accurate prediction of the response of a patient diagnosed with melanoma to targeted pharmacotherapy.

### SUMMARY OF THE INVENTION

Drug response between individuals differs. Drugs can work more or less efficient; but can also induce adverse drug reactions, toxicity and side effects.

The present invention provides methods and devices that enable the determination of the response of a patient diagnosed with melanoma to an immunotherapeutic antibody directed against CTLA-4 by measuring phosphatase activity or phosphatase activity and kinase activity of a sample obtained from a blood sample of said patient. The response of the patient can be determined by adverse drug reactions, toxicity and/or side effects.

The present invention further provides in a method for predicting the response of a patient diagnosed with melanoma cancer, to an immunotherapeutic antibody directed against CTLA-4, comprising the steps of:
(a) measuring the phosphatase activity of a sample, obtained from said patient diagnosed with melanoma, by contacting said sample with at least one protein phosphatase substrate, thereby providing a phosphorylation profile of said sample, said phosphorylation profile comprising the phosphorylation levels of phosphorylation sites present in at least the peptide markers as defined by SEQ ID NOs: 139 - 148; wherein said sample is obtained from a blood sample of said patient diagnosed with melanoma; and,
(b) determining from said phosphorylation profile the response of said patient to said immunotherapeutic antibody directed against CTLA-4.

The method according to the invention further provides that said blood sample comprises peripheral blood mononuclear cells.

The method according to the invention further provides that said medicament is Ipilimumab.

The method according to the invention further provides that said phosphorylation profiles comprise the phosphorylation levels of phosphorylation sites present in at least the peptide markers defined by SEQ ID NOs: 139 - 148 and at least 20 further peptide makers as listed in Table 2.

The method according to the invention further provides that said phosphorylation profiles comprise the phosphorylation levels of phosphorylation sites present in all the peptide markers as listed in Table 2.

The method according to the invention further provides measuring the kinase activity of said sample, by contacting said sample with at least one protein kinase substrate and wherein said phosphorylation profile further comprises the phosphorylation levels of phosphorylation sites present in at least 10 peptide markers as listed in Table 1 and/or Table 3.

The method according to the invention further provides that step (b) is replaced by a step (c) calculating a classifier parameter from said phosphorylation profile; and a step (d) determining the response of said patient to said immunotherapeutic antibody directed against CTLA-4 on the basis of said classifier parameter.

The method according to the invention further provides that said classifier parameter indicates said patient being a good responder to said immunotherapeutic antibody directed against CTLA-4if said classifier parameter is above a first predetermined threshold level, and wherein said classifier parameter indicates said patient being a poor responder to said immunotherapeutic antibody directed against CTLA-4if said classifier parameter is below a second predetermined threshold level.

The method according to the invention further provides that step (b) is replaced by a step (e) comparing said phosphorylation profile to a first and a second reference phosphorylation profile; said first reference phosphorylation profile being representative for a good responder to said immunotherapeutic antibody directed against CTLA-4and said second reference phosphorylation profile being representative for a poor responder to said immunotherapeutic antibody directed against CTLA-4; and a step (f) determining response of said patient to said immunotherapeutic antibody directed against CTLA-4on the basis of the comparison of said phosphorylation profile with said first and said second reference phosphorylation profile.

The method according to the invention further provides that said phosphorylation profile or said classifier parameter indicates good response, poor response or undetermined response of said patient to said immunotherapeutic antibody directed against CTLA-4.

The method according to the invention further provides that said melanoma is an irresectable stage Illc or IV melanoma.

The method according to the invention further provides that from the measurements in step (a) the toxicity of said immunotherapeutic antibody directed against CTLA-4in said patient is determined. Adverse toxicological effects of medicaments are related to the physicochemical characteristics of a compound and its effects on cellular organelles, membranes, metabolic and on-target or off-target cell signalling pathways. Off-target refers to adverse effects as a result of modulation of other targets; these may be related biologically or totally unrelated to the target of interest. It is imperative that the toxicological pathologist use the toxicological and biologic and patient derived data such as cell signalling data of patient derived materials to test whether toxicity is chemical-based and induces off-target effects. Studying off-target-based effects are important as an aid in individualized human risk assessment of medicaments.

In a third aspect the present invention provides in a computer program product for use in conjunction with a computer having a processor and a memory connected to the processor, said computer program product comprising a computer readable storage medium having a computer program mechanism encoded thereon, wherein said computer program mechanism may be loaded into the memory of said computer and cause said computer to carry out the method according to the invention.

These and further aspects and embodiments are described in the following sections and in the claims.

### BRIEF DESCRIPTION OF FIGURES

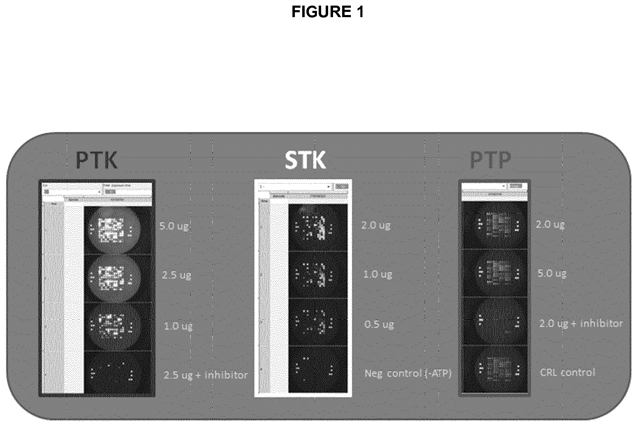

**Figure 1** shows that patient-derived blood profiling based on protein tyrosine kinase (PTK), serine/threonine protein kinase (STK/TTK) and protein tyrosine phosphatase (PTP) assays is sensitive and provides high signals for only 1-2 µg of protein in all assays.

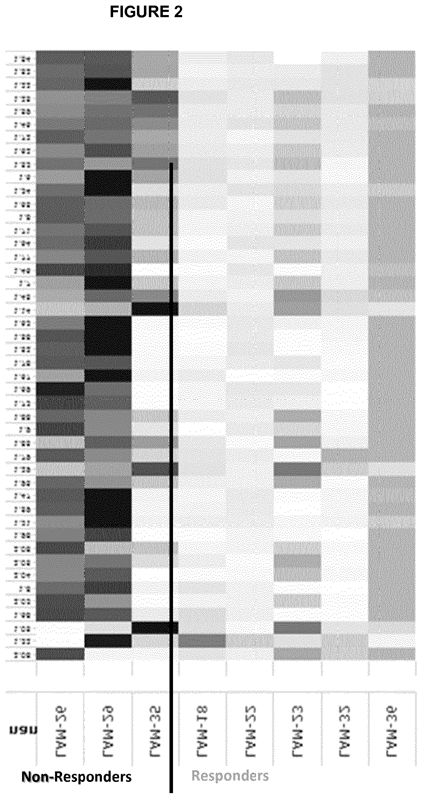

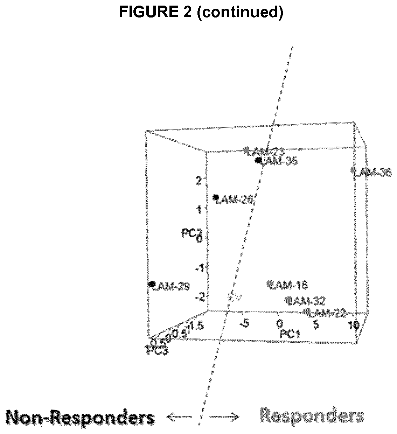

**Figure 2** shows the phosphatase activity profiling of ten patient-derived PBMC samples and the profiles correlation to response. The intensity of the phosphatase activities correlates almost perfectly with the clinical response (87.5%). Hence, the method according to the invention allows a good prediction of the treatment outcome of patients treated with Ipilimumab.

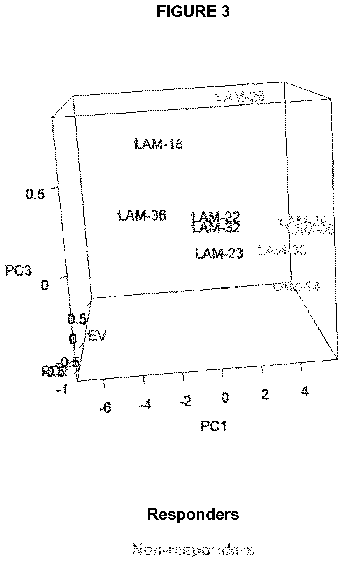

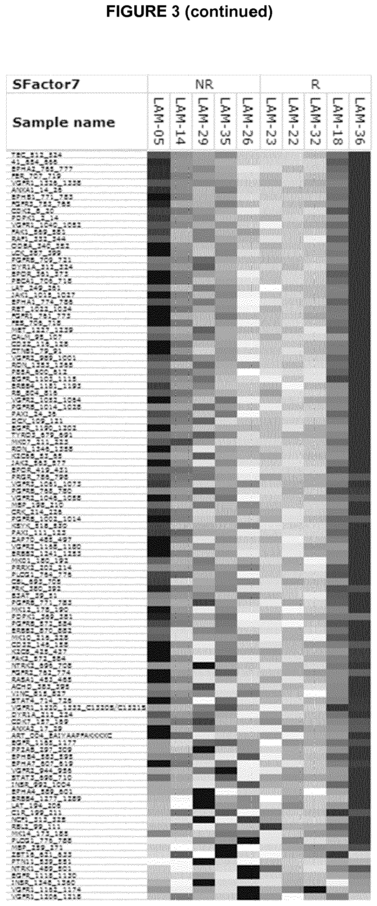

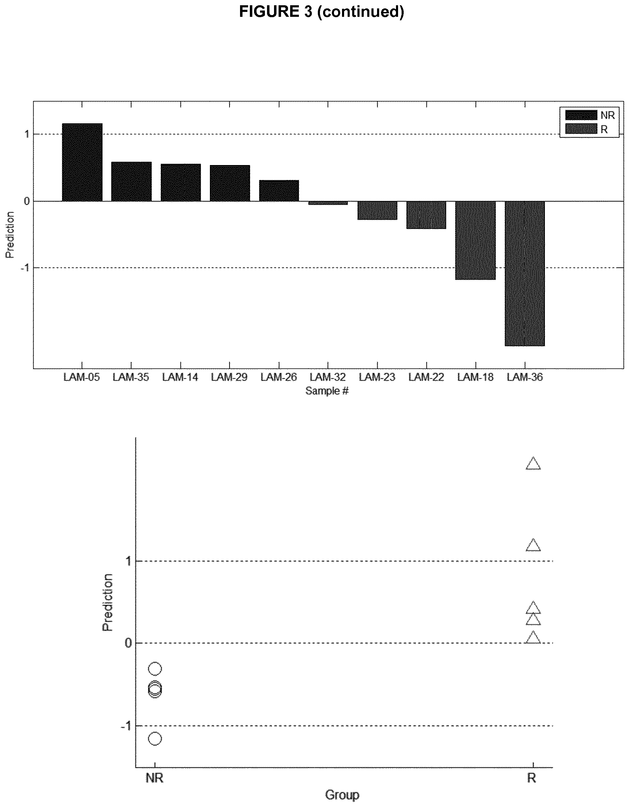

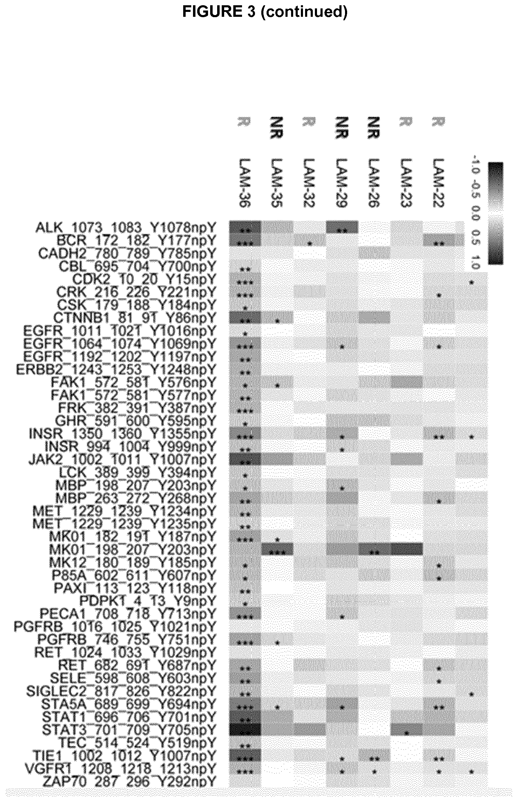

**Figure 3** shows the kinase activity profiling of ten patient-derived PBMC samples and the profiles correlation to response. The intensity of the kinase activities correlates almost perfectly with the clinical response. Hence, the method according to the invention allows a good prediction of the treatment outcome of patients treated with Ipilimumab.

### DETAILED DESCRIPTION OF THE INVENTION

Before the present method and devices used in the invention are described, it is to be understood that this invention is not limited to particular methods, components, or devices described, as such methods, components, and devices may, of course, vary. It is also to be understood that the terminology used herein is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

In this specification and the appended claims, the singular forms "a", "an", and "the" include plural references unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The terms "comprising", "comprises" and "comprised of" also include the term "consisting of".

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

The present invention provides methods and devices that enable the determination of the response of a patient diagnosed with melanoma to an immunotherapeutic antibody directed against CTLA-4 by measuring phosphatase activity or phosphatase activity and kinase activity of a sample, obtained from a blood sample from said patient diagnosed with melanoma. The present invention further shows how the method and devices can be used to predict the response of patients diagnosed with melanoma to a specific medicament. The method of the present invention therefore adds to the existing assays currently used to select therapies in melanoma patients.

In one embodiment of the present invention, methods are provided wherein the response of a patient diagnosed with melanoma to an immunotherapeutic antibody directed against CTLA-4 is determined by measuring the phosphatase activity and the kinase activity, preferably the protein kinase activity. For purposes of the present invention, and as used herein the term "kinase activity" or "protein kinase activity" refer to the formation of reaction product(s) by a certain amount of kinase or protein kinase acting on a substrate during the course of the assay.

Protein kinase activity is referred to as the activity of protein kinases. A protein kinase is a generic name for all enzymes that transfer a phosphate to a protein. About two percent of the human genome contains transcription information for the formation of protein kinases. Currently, there are about 518 known different protein kinases. However, because three to four percent of the human genome is a code for the formation of protein kinases, there may be many more separate kinases in the human body.

Phosphatase activity is referred to as the activity of protein phosphatases. A phosphatase is a generic name for all enzymes able to remove a phosphate group from a substrate by hydrolysing phosphoric acid monoesters into a phosphate ion and a molecule with a free hydroxyl group. This action is directly opposite to that of phosphorylases and kinases, which attach phosphate groups to their substrates by using energetic molecules like ATP. Protein phosphatases (PPs) are the primary effectors of dephosphorylation and can be grouped into three main classes based on sequence, structure and catalytic function. The largest class of PPs is the phosphoprotein phosphatase (PPP) family comprising PP1, PP2A, PP2B, PP4, PP5, PP6 and PP7, and the protein phosphatase Mg²⁺- or Mn²⁺-dependent (PPM) family, composed primarily of PP2C. The protein Tyrosine phosphatase (PTP) super-family forms the second group, and the aspartate-based protein phosphatases the third.

A protein kinase is a kinase enzyme that modifies other proteins by covalently coupling phosphate groups to them. This process or activity is also referred to as phosphorylation. Phosphorylation can therefore be regarded as the process of the addition of a phosphate group to a substrate. Phosphorylation usually results in a functional change of the substrate by changing kinase activity, cellular location, or association with other proteins. Up to 30 percent of all proteins may be modified by kinase activity, and kinases are known to regulate the majority of cellular pathways, especially those involved in signal transduction, the transmission of signals within the cell. The chemical activity of a kinase involves removing a phosphate group from **ATP** or GTP and covalently attaching it to amino acids such as serine, threonine, tyrosine, histidine, aspartic acid and/or glutamic acid that have a free hydroxyl group. Most known kinases act on both serine and threonine, others act on tyrosine, and a number act on all serine, threonine and tyrosine. The protein kinase activity monitored with the method of the present invention is preferably directed to protein kinases acting towards serine, threonine and/or tyrosine, preferably acting on both serine and threonine, on tyrosine or on serine, threonine and tyrosine and more preferably the method of the present invention if preferably directed to protein kinases acting towards serine and threonine.

In another embodiment of the present invention, methods are provided wherein the response of a patient diagnosed with melanoma to an immunotherapeutic antibody directed against CTLA-4 is determined by measuring the phosphatase activity, preferably the protein phosphatase activity. For purposes of the present invention, and as used herein the term "phosphatase activity" or "protein phosphatase activity" refer to the formation of reaction product(s) by a certain amount of phosphatase or protein phosphatase acting on a substrate during the course of the assay.

A protein phosphatase is a phosphatase enzyme that modifies other proteins by enzymatically removing phosphate groups from them. This process or activity is also referred to as dephosphorylation. Dephosphorylation can therefore be regarded as the process of the removing a phosphate group from a substrate. Dephosphorylation usually results in a functional change of the substrate by changing phosphatase activity, cellular location, or association with other proteins. Up to 30% of all proteins may be modified by phosphatase activity, and phosphatases are known to regulate the majority of cellular pathways, especially those involved in signal transduction, the transmission of signals within the cell. The activity of a phosphatase involves removing a phosphate group from amino acids such as serine, threonine, tyrosine, histidine, aspartic acid and/or glutamic acid that have a free hydroxyl group. Most known phosphatases act on both serine and threonine, others act on tyrosine, and a number act on all serine, threonine and tyrosine. The protein phosphatase activity that can be monitored with the tools provided by the present invention is preferably directed to protein phosphatases acting towards tyrosines.

Protein kinases are distinguished by their ability to phosphorylate substrates on discrete sequences. These sequences have been determined by sequencing the amino acids around the phosphorylation sites and are usually distinct for each protein kinase. The recognition sequence on each substrate is specific for each kinase catalyst.

Protein phosphatases are distinguished by their ability to dephosphorylate substrates on discrete sequences. These sequences can be determined by sequencing the amino acids around the dephosphorylation sites and are usually distinct for each type of protein phosphatase.

In another embodiment of the present invention, methods are provided wherein the response of a patient diagnosed with melanoma to an immunotherapeutic antibody directed against CTLA-4 is determined by measuring both kinase and phosphatase activity, preferably protein kinase and protein phosphatase activity. Because protein kinases and protein phosphatases have profound effects on a cell, their activity is highly regulated. Kinases and phosphatases are turned on or off by for instance phosphorylation, by binding of activator proteins or inhibitor proteins, or small molecules, or by controlling their location in the cell relative to their substrates. Deregulated kinase and/or phosphatase activity is a frequent cause of disease, particularly cancer, where kinases regulate many aspects that control cell growth, movement and death. Therefore monitoring the protein kinase and/or protein phosphatase activity in tissues can be of great importance and a large amount of information can be obtained when comparing the kinase and/or phosphatase activity of different tissue samples.

As described in the present invention, the inventors have surprisingly found that the response of a patient diagnosed with melanoma to an immunotherapeutic antibody directed against CTLA-4 can be predicted and/or determined on the basis of the measurement of the phosphatase activity or the phosphatase and kinase activity, preferably protein phosphatase activity or protein phosphatase and protein kinase activity, of a sample obtained from blood taken from said patient diagnosed with melanoma. The methods according to present invention enable to provide information regarding the efficacy of the an immunotherapeutic antibody directed against CTLA-4 treatment, and more specifically provide an early determination of the most suited treatment of the melanoma patient.

The measurement of the kinase and/or phosphatase activity is performed by contacting a sample from a patient diagnosed melanoma with one or more kinase and/or phosphatase substrates, preferably protein kinase and/or protein phosphatase substrates, thereby generating one or more phosphorylation profile(s).

Said protein kinase and/or protein phosphatase substrates as used herein, are preferably peptides, proteins or peptide mimetics. The protein kinase substrates each comprise, preferably one or more, phosphorylation sites that can be phosphorylated by the protein kinases or dephosphorylated by the protein phosphatases present in the sample. Therefore, exposure of a protein kinase substrate to a sample comprising a protein kinase results in the phosphorylation of one or more of the phosphorylation sites of the protein kinase substrate. Alternatively, exposure of a protein phosphatase substrate to a sample comprising a protein phosphatase results in the dephosphorylation of one or more of the phosphorylation sites of the protein phosphatase substrate. This phosphorylation and/or dephosphorylation activity can be measured using techniques known in the art. Therefore, during the measurement method the kinase enzymes present in the sample will phosphorylate, preferably one or more, of the phosphorylation sites on one or more protein kinase substrate and/or the phosphatase enzymes present in the sample will dephosphorylate, preferably one or more, of the phosphorylation sites on one or more protein phosphatase substrate. The inventors have observed essential differences between kinase and/or phosphatase activity of melanoma tumours having a different response to an immunotherapeutic antibody directed against CTLA-4. Consequently, the inventors have observed that the kinases and/or phosphatases present in a sample from patients suffering from melanoma will phosphorylate protein kinase substrates and/or dephosphorylate protein phosphatase substrates differently depending on the response to an immunotherapeutic antibody directed against CTLA-4 with which the patient is envisaged to be treated or is being treated. Phosphorylation signals differ between the samples, resulting in phosphorylation patterns that differ depending on response to an immunotherapeutic antibody directed against CTLA-4. Combining the phosphorylation profiles based on kinase and phosphatase activity provides an even more accurate prediction to the response to targeted pharmacotherapy.

For purposes of the present invention, and as used herein the term "pharmacotherapy", or "pharmacotherapeutics" or "drug treatment" refers to the use of a pharmaceutical drug, also referred to as medicine or medicament wherein said pharmacotherapy is intended for use in the diagnosis, cure, mitigation, treatment, or prevention of disease.

In a further embodiment, the present invention therefore provides in a method for predicting the response of a patient diagnosed with melanoma cancer, to an immunotherapeutic antibody directed against CTLA-4, comprising the steps of:
(a) measuring the phosphatase activity of a sample, obtained from said patient diagnosed with melanoma, by contacting said sample with at least one protein phosphatase substrate, thereby providing a phosphorylation profile of said sample, said phosphorylation profile comprising the phosphorylation levels of phosphorylation sites present in at least the 10 peptide markers as defined by SEQ ID Nos: 139 - 148; and,
(b) determining from said phosphorylation profile the response of said patient to said immunotherapeutic antibody directed against CTLA-4.

In a preferred embodiment, the present invention therefore provides in a method for predicting the response of a patient diagnosed with melanoma cancer, to an immunotherapeutic antibody directed against CTLA-4, comprising the steps of:
(a) measuring the kinase and phosphatase activity of a sample, obtained from said patient diagnosed with melanoma, by contacting said sample with at least one protein kinase substrate and at least one protein phosphatase substrate, thereby providing two phosphorylation profiles of said sample, said phosphorylation profiles comprising the phosphorylation levels of phosphorylation sites present in at least the 10 peptide markers as defined by SEQ ID NOs: 139 - 148, and further comprises the phosphorylation levels of phosphorylation sites present in at least 10 peptide markers as listed in Table 1 and/or Table 3; and,
(b) determining from said phosphorylation profiles the response of said patient to said immunotherapeutic antibody directed against CTLA-4.

In said preferred embodiment of present invention, a single sample obtained from a patient diagnosed with melanoma cancer is split into a first part that is used for the measurement of the kinase activity of said sample while a second part of the sample is used for the measurement of the phosphatase activity of said sample. These measurements provide two individual phosphorylation profiles of said sample: one comprising the phosphorylation levels of phosphorylation sites present in the at least 10 peptide markers as defined in SEQ ID NOs: 139 - 148, and another comprising the phosphorylation levels of phosphorylation sites present in at least 10 peptide markers as listed in Table 1 and/or Table 3. The combination of these two different phosphorylation profiles improves the determination of the response of said patient to an immunotherapeutic antibody directed against CTLA-4 compared to the use of only one phosphorylation profile.

It is clear that effects of an immunotherapeutic antibody directed against CTLA-4 can be monitored using the method according to the invention as described herein. The immunotherapeutic antibody directed against CTLA-4 affects the degree of inhibition, the potency and/or the selectivity of the phosphatases in the sample. More peptide inhibition is caused by the larger effect of the immunotherapeutic antibody directed against CTLA-4 on the phosphatases in the sample and therefore the drug is less selective. Also an increased peptide inhibition would lead to a larger amount of normal tissues being affected by the drug, making the drug less tumour tissue specific.

As referred to in the present application melanoma regards a specific type of skin cancer which forms from melanocytes (pigment-containing cells in the skin). While other types of skin cancer (e.g. basal cell cancer (BCC) and squamous cell cancer (SCC)) are more common, melanoma is considered to be much more dangerous if it is not found in the early stages. It causes the majority (75%) of deaths related to skin cancer. Globally, in 2012, melanoma occurred in 232,000 people and resulted in 55,000 deaths.

As used in the present invention, the term "sample" refers to a sample obtained from an organism (patient) such as human or from components (e.g. tissue or cells) of such an organism. Blood is considered a specialized form of connective tissue. Surface skin biopsies, punch biopsies or excisional biopsies are considered unpleasant for the patient and can result in the formation of scar tissue. Therefore, other samples than melanoma tumour tissue samples are preferred. In the present invention, said sample is obtained from a patient diagnosed with melanoma and is derived from the blood of said patient. Preferably, said blood sample comprises peripheral blood monocytes (PBMCs).

Said sample is preferably a fresh or a fresh frozen sample.

More preferably, said sample refers to a lysate of blood-derived PBMCs, which are preferably isolated by Ficoll-Isopaque density centrifugation or by any methods known in the art.

Alternatively said sample may be derived from a melanoma tumour sample tissue or a metastasis thereof. Said sample may be obtained from a melanoma tumour sample tissue or a metastasis thereof that has been cultured in vitro for a limited period of time. Said sample may be obtained from specific melanoma cell lines and in particular cell lysates thereof.

In a preferred embodiment of the present invention said sample is a sample that has undergone a preparation step prior to the steps according to the method of the present invention. Preferably said preparation step is a step where the protein kinases and/or protein phosphatases present in said sample are released from the tissue by lysis. Additionally the kinases and/or phosphatases in the sample may be stabilized, maintained, enriched or isolated, and the measurement of the kinase and/or phosphatase activity as performed in step (a) occurs on the enriched or isolated protein kinase and/or protein phosphatase sample. By first enriching protein kinases and/or protein phosphatases in the sample or isolating protein kinases and/or protein phosphatases from the sample the subsequent measurement of the kinase and/or phosphatase activity will occur in a more efficient and reliable manner. Also the clarity and intensity of the obtained phosphorylation signal will be increased as certain contaminants are being removed during the enriching or isolating step.

As used in the present invention, the term "phosphorylation profile" refers to a data set representative for the phosphorylation levels of, preferably one or more, phosphorylation sites present on the protein kinase and/or protein phosphatase substrates. When measuring the kinase and/or phosphatase activity of a sample by contacting said sample with protein kinase and/or protein phosphatase substrates a specific phosphorylation profile is obtained. The phosphorylation profile is generated by the phosphorylation of the protein kinase and/or protein phosphatase substrates with the protein kinases and/or protein phosphatases present in the sample and it comprises the level of phosphorylation of the phosphorylation sites present on the protein kinase and/or protein phosphatase substrates used. A phosphorylation profile can thus be generated when using at least one protein phosphatase substrate in different test conditions such as for example by comparing the phosphorylation of a sample on one peptide or protein (phosphatase substrate) in the presence and absence of a phosphatase modulating compound or medicament. More frequently phosphorylation profiles of a sample will be measured using several protein kinase and/or protein phosphatase substrates in the same or sequentially carried out experiments. Preferably, the present invention determines tyrosine, serine and threonine kinase and/or tyrosine phosphatase activity levels or profiles.

It should be noted that a person skilled in the art will appreciate that the methods of the present invention can use phosphorylation profiles as a basis for determining the predicting the response to an immunotherapeutic antibody directed against CTLA-4 of a patient suffering from melanoma.

It should be noted that for the measurement of the protein kinase activity, ATP or any other phosphate source needs to be added to the sample when it is contacted with the protein kinase substrates. The presence of ATP will lead to a phosphorylation of the protein kinase substrates. Alternatively, the phosphorylation of the protein kinase substrates can be performed in the absence of exogenous ATP. When no ATP is added during the incubation of the sample with the protein kinase substrates, the endogenous ATP, the ATP naturally present in the sample, will act as the primary source of ATP.

The phosphorylation level of each of the protein kinase substrates can be monitored using any method known in the art. The response of the protein kinase substrates is determined using a detectable signal, said signal resulting from the interaction of the sample with the protein kinase substrates or by for instance measuring mass differences using mass spectrometry. In determining the interaction of the sample with the protein kinase substrates the signal is the result of the interaction of the phosphorylated substrates with a molecule capable of binding to the phosphorylated substrates. This binding can be detected by e.g. surface plasmon resonance or by the molecule being detectably labelled. For the latter, the molecule that specifically binds to the substrates of interest (e.g. antibody or polynucleotide probe) can be detectably labelled by virtue of containing an atom (e.g. radionuclide), molecule (e.g. fluorescein), or enzyme or particle or complex that, due to a physical or chemical property, indicates the presence of the molecule. A molecule may also be detectably labelled when it is covalently bound to or otherwise associated with a "reporter" molecule (e.g. a biomolecule such as an enzyme) that acts on a substrate to produce a detectable atom, molecule or other complex.

The phosphorylation level of each of the protein phosphatase substrates can be monitored using typical methods known in the art. The dephosphorylation of the protein phosphatase substrates, provided with the tyrosine residues, result in a detectable signal. This signal can be either attributed to a reaction of the substrates with antibodies, but also other measurement methods are available such as for instance measuring mass differences using mass spectrometry or the direct measurement of the dephosphorylation since the product of the reaction contains a nitrophenol moiety which absorbs at 405nm.

In determining the interaction of the sample with the protein phosphatase substrates, the signal is the result of the interaction of the substrates with a detectably labelled molecule capable of binding to the substrates subjected to the dephosphorylation. For the latter, the molecule that specifically binds to the substrates of interest (e.g., antibody) can be detectably labelled by virtue of containing an atom (e.g., radionuclide), molecule (e.g., fluorescein), or enzyme or particle or complex that, due to a physical or chemical property, indicates the presence of the molecule. A molecule may also be detectably labelled when it is covalently bound to or otherwise associated with a "reporter" molecule (e.g., a biomolecule such as an enzyme) that acts on a substrate to produce a detectable atom, molecule or other complex.

Detectable labels suitable for use in the present invention include any composition detectable by spectroscopic, photochemical, biochemical, immunochemical, electrical, optical or chemical means. Labels useful in the present invention include biotin for staining with labelled avidin or streptavidin conjugate, magnetic beads (e.g. Dynabeads'), fluorescent dyes (e.g. fluorescein, fluorescein-isothiocyanate (FITC), Texas red, rhodamine, green fluorescent protein, enhanced green fluorescent protein and related proteins with other fluorescence emission wavelengths, lissamine, phycoerythrin, Cy2, Cy3, Cy3.5, Cy5, Cy5.5, Cy7, FluorX [Amersham], SYBR Green I & II [Molecular Probes], and the like), radiolabels (e.g. 3H,125I, 35S, 14C, or 32P), enzymes (e.g. luciferases, hydrolases, particularly phosphatases such as alkaline phosphatase, esterases and glycosidases, or oxidoreductases, particularly peroxidases such as horse radish peroxidase, and the like), substrates, cofactors, chemilluminescent groups, chromogenic agents, and colorimetric labels such as colloidal gold or coloured glass or plastic (e. g. polystyrene, polypropylene, latex, etc.), protein particles or beads. In particular, all detectable labels well known to those skilled in the art may be used as detectable labels for use in the present invention.

Means of detecting such labels are well known to those of skill in the art. Thus, for example, chemiluminescent and radioactive labels may be detected using photographic film or scintillation counters, and fluorescent markers may be detected using a photodetector to detect emitted light (e.g. as in fluorescence-activated cell sorting). Enzymatic labels are typically detected by providing the enzyme with a substrate and detecting a coloured reaction product produced by the action of the enzyme on the substrate. Colorimetric labels are detected by simply visualizing the coloured label. Thus, for example, where the label is a radioactive label, means for detection include a scintillation counter, photographic film as in autoradiography, or storage phosphor imaging. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge coupled devices (CCDs) or photomultipliers and the like. Similarly, enzymatic labels may be detected by providing the appropriate substrates for the enzyme and detecting the resulting reaction product. Also, simple colorimetric labels may be detected by observing the colour associated with the label. Fluorescence resonance energy transfer has been adapted to detect binding of unlabeled ligands, which may be useful on arrays.

In a particular embodiment of the present invention the response of the protein kinase and/or protein phosphatase substrates to the sample is determined using detectably labelled antibodies; more in particular fluorescently labelled antibodies. In those embodiments of the invention where the substrates consist of protein kinase substrates, the response of the protein kinase substrates is determined using fluorescently labelled anti-phosphotyrosine antibodies, fluorescently labelled anti-phosphoserine or fluorescently labelled anti-phosphothreonine antibodies. The use of fluorescently labelled anti-phosphotyrosine antibodies or fluorescently labelled anti-phosphoserine or fluorescently labelled anti-phosphothreonine antibodies in the method of the present invention, allows real-time or semi real-time determination of the protein kinase activity and accordingly provides the possibility to express the protein kinase activity as the initial velocity of protein kinase derived from the activity over a certain period of incubation of the sample on the substrates. In those embodiments of the invention where the substrates consist of protein phosphatase substrates, the The response of the protein phosphatase substrates is determined using fluorescently labelled anti-nitrotyrosine antibodies as described in EP application 15168371.1 (which is enclosed herein by reference). The use of fluorescently labelled anti-nitrotyrosine antibodies in the method of the present invention, allows real-time or semi real-time determination of the phosphatase activity and accordingly provides the possibility to express the phosphatase activity as the initial velocity of phosphatase derived from the activity over a certain period of incubation of the sample on the substrates.

Moreover, the measurement of the phosphatase activity or the phosphatase and kinase activity of said sample occurs by contacting said sample with at least the 10 the peptide markers as defined by SEQ ID NOs: 139 - 148, or with at least the 10 the peptide markers as defined by SEQ ID NOs: 139 - 148 and in at least 10 peptide markers as listed Table 1 and/or Table 3.

In another embodiment according to the present invention, the phosphorylation profiles comprise the phosphorylation levels of phosphorylation sites present in at least the 10 peptide markers as defined by SEQ ID NOs: 139 - 148, and at least 10 of the peptide markers Table 1 and/or Table 3. Preferably phosphorylation levels will be studied of phosphorylation sites present in at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 of the peptide markers listed in Table 2 with the provision that at least 10 of those peptides are defined by SEQ ID NOs: 139 - 148, and preferably at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57 ,58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140 or 141 of the peptide markers listed in Table 1 and/or Table 3.

The term "peptide markers" in the context of the present invention refers to the fact that the peptides as listed in Table 1, Table 3 and/or Table 2 can be preferably used according to the methods of the present invention to measure the phosphorylation levels of phosphorylation sites of said markers in samples. The phosphorylation levels of the individual phosphorylation sites present in said markers may be measured and compared in different ways. Therefore the present invention is not limited to the use of peptides identical to any of these peptide markers as listed in Table 1, Table 3 and/or Table 2 as such. The skilled person may easily on the basis of the peptide markers listed in Table 1, Table 3 and/or Table 2 design variant peptides compared to the specific peptides in said Tables and use such variant peptides in a method for measuring phosphorylation levels of phosphorylation sites common to said peptide markers as listed in Table 1, Table 3 and/or Table 2. These variant peptides may have one or more (2, 3, 4, 5, 6, 7, etc.) amino acids more or less than the given peptides and may also have amino acid substitutions (preferably conservative amino acid substitutions) as long as these variant peptides retain at least one or more of the phosphorylation sites of said original peptides as listed in said tables. Further the skilled person may also easily carry out the methods according to the present invention by using proteins (full length or N- or C-terminally truncated) comprising the amino acid regions of the "peptide markers" listed in Table 1, Table 3 and/or Table 2 as sources for studying the phosphorylation of sites present in the amino acid regions of the peptides listed in Table 1, Table 3 and/or Table 2. Also the skilled person may use peptide mimetics.

The protein kinase and/or protein phosphatase substrates as used in the methods described herein, are meant to include peptides, proteins or peptide mimetics comprising, preferably one or more, of the phosphorylation sites of the peptide markers of Table 1, Table 3 and/or Table 2. Said one or more phosphorylation sites are specifically phosphorylated by the protein kinases and/or protein phosphatases present in the sample thereby providing a phosphorylation profile. More preferably the protein kinase and/or protein phosphatase substrates (peptides, proteins or peptide mimetics) as used in the method of the present invention comprise at least the 10 peptide markers as defined by SEQ ID NOs: 139 - 148, and preferably at least 10 peptide markers as listed in Table 1 and/or Table 3. More particularly said protein kinase substrates represent the one or more phosphorylation sites present in at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 of the peptide markers listed in Table 2 with the provision that 10 of these peptide markers are as defined by SEQ ID NOs: 139 - 148, and preferably at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57 ,58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140 or 141 of the peptide markers listed in Table 1 and/or Table 3, and/or. In a more preferred embodiment the protein kinase and/or protein phosphatase substrates comprise or consist of, preferably one or more, phosphorylation sites present in all of the peptide markers listed in Table 1 and/or Table 3.

The inventors have found that especially the peptide markers with SEQ ID NO 1 up to SEQ ID NO 10 as listed in Table 1 and with SEQ ID NO 139 up to SEQ ID NO 148 as listed in Table 2 enable the prediction of pharmacotherapy response in melanoma patients, in particular patients treated with Ipilimumab.

The inventors have also found that especially the peptide markers with SEQ ID NO 105, 17, 99, 94, 128, 24, 11, 1, 72, 16, 69, 187, 45, 22, 189, 48, 80, 55, 81, 124, 188, 49 and 60 as listed in Table 1 and/or Table 3 enable the prediction of pharmacotherapy response in melanoma patients, in particular patients treated with Pembrolizumab.

The peptide markers as listed in Table 1, Table 3 and/or Table 2 could serve as an accurate early indicator for therapeutic response in a mammalian subject to measure the effectiveness of candidate melanoma inhibitory agents.

**Table 1: list of 141 peptide markers comprising phosphorylation sites used for determining the kinase activity, their sequence and SEQ ID NO. The name of the peptide markers refers to the associated proteins and to the start and the end position of the amino acid sequence.**

| **SEQ ID NO** | **Name** | **Sequence** |
|---|---|---|
| 1 | CDK7_157_169 | GLAKSFGSPNRAY |
| 2 | TYRO3_679_691 | KIYSGDYYRQGCA |
| 3 | PRGR_786_798 | EQRMKESSFYSLC |
| 4 | PDPK1_369_381 | DEDCYGNYDNLLS |
| 5 | VGFR1_1326_1338 | DYNSVVLYSTPPI |
| 6 | FGFR1_761_773 | TSNQEYLDLSMPL |
| 7 | K2C6B_53_65 | GAGFGSRSLYGLG |
| 8 | PRRX2_202_214 | WTASSPYSTVPPY |
| 9 | VGFR3_1061_1073 | DIYKDPDYVRKGS |
| 10 | PGFRB_709_721 | RPPSAELYSNALP |
| 11 | FGFR2_762_774 | TLTTNEEYLDLSQ |
| 12 | EGFR_1165_1177 | ISLDNPDYQQDFF |
| 13 | VGFR1_1320_1332_C1320S/C1321S | SSSPPPDYNSVVL |
| 14 | RON_1353_1365 | YVQLPATYMNLGP |
| 15 | PGFRB_768_780 | SSNYMAPYDNYVP |
| 16 | RET_1022_1034 | TPSDSLIYDDGLS |
| 17 | ANXA1_14_26 | IENEEQEYVQTVK |
| 18 | FGFR3_753_765 | TVTSTDEYLDLSA |
| 19 | ODBA_340_352 | DDSSAYRSVDEVN |
| 20 | PDPK1_2_14 | ARTTSQLYDAVPI |
| 21 | TEC_512_524 | RYFLDDQYTSSSG |
| 22 | FER_707_719 | RQEDGGVYSSSGL |
| 23 | PGFRB_1002_1014 | LDTSSVLYTAVQP |
| 24 | CBL_693_705 | EGEEDTEYMTPSS |
| 25 | RB_804_816 | IYISPLKSPYKIS |
| 26 | VGFR2_1052_1064 | DIYKDPDYVRKGD |
| 27 | FAK1_569_581 | RYMEDSTYYKASK |
| 28 | MET_1227_1239 | RDMYDKEYYSVHN |
| 29 | NTRK2_696_708 | GMSRDVYSTDYYR |
| 30 | DYR1A_312_324 | CQLGQRIYQYIQS |
| 31 | CD3Z_146_158 | STATKDTYDALHM |
| 32 | DCX_109_121 | GIVYAVSSDRFRS |
| 33 | PGFRB_771_783 | YMAPYDNYVPSAP |
| 34 | LCK_387_399 | RLIEDNEYTAREG |
| 35 | JAK1_1015_1027 | AIETDKEYYTVKD |
| 36 | VGFR2_1168_1180 | AQQDGKDYIVLPI |
| 37 | MK07_211_223 | AEHQYFMTEYVAT |
| 38 | MBP_198_210 | ARTAHYGSLPQKS |
| 39 | B3AT_39_51 | TEATATDYHTTSH |
| 40 | CD3Z_116_128 | KDKMAEAYSEIGM |
| 41 | ANXA2_17_29 | HSTPPSAYGSVKA |
| 42 | EGFR_1190_1202 | STAENAEYLRVAP |
| 43 | CALM_95_107 | KDGNGYISAAELR |
| 44 | PECA1_706_718 | KKDTETVYSEVRK |
| 45 | EPOR_361_373 | SEHAQDTYLVLDK |
| 46 | EPHA2_765_777 | EDDPEATYTTSGG |
| 47 | STAT4_714_726 | PSDLLPMSPSVYA |
| 48 | FES_706_718 | REEADGVYAASGG |
| 49 | EPHB1_771_783 | DDTSDPTYTSSLG |
| 50 | PP2AB_297_309 | EPHVTRRTPDYFL |
| 51 | CRK_214_226 | GPPEPGPYAQPSV |
| 52 | LAT_249_261 | EEGAPDYENLQEL |
| 53 | VGFR2_989_1001 | EEAPEDLYKDFLT |
| 54 | FAK2_572_584 | RYIEDEDYYKASV |
| 55 | CDK2_8_20 | EKIGEGTYGVVYK |
| 56 | CTNB_79_91 | VADIDGQYAMTRA |
| 57 | ERBB2_1241_1253 | PTAENPEYLGLDV |
| 58 | RON_1346_1358 | SALLGDHYVQLPA |
| 59 | PGFRB_1014_1028 | PNEGDNDYIIPLPDP |
| 60 | EPHA1_774_786 | LDDFDGTYETQGG |
| 61 | ZAP70_485_497 | ALGADDSYYTARS |
| 62 | ERBB4_1181_1193 | QALDNPEYHNASN |
| 63 | 41_654_666 | LDGENIYIRHSNL |
| 64 | MK01_180_192 | HTGFLTEYVATRW |
| 65 | VGFR2_1046_1058 | DFGLARDIYKDPD |
| 66 | PAXI_24_36 | FLSEETPYSYPTG |
| 67 | RAF1_332_344 | PRGQRDSSYYWEI |
| 68 | ACHD_383_395 | YISKAEEYFLLKS |
| 69 | FRK_380_392 | KVDNEDIYESRHE |
| 70 | KSYK_518_530 | ALRADENYYKAQT |
| 71 | RASA1_453_465 | TVDGKEIYNTIRR |
| 72 | PLCG1_764_776 | IGTAEPDYGALYE |
| 73 | K2C8_425_437 | SAYGGLTSPGLSY |
| 74 | ART_004_EAIYAAPFAKKKXC | EAIYAAPFAKKK |
| 75 | EPOR_419_431 | ASAASFEYTILDP |
| 76 | EGFR_1103_1115 | GSVQNPVYHNQPL |
| 77 | JAK2_563_577 | VRREVGDYGQLHETE |
| 78 | MK10_216_228 | TSFMMTPYVVTRY |
| 79 | DYR1A_212_224 | KHDTEMKYYIVHL |
| 80 | P85A_600_612 | NENTEDQYSLVED |
| 81 | PAXI_111_123 | VGEEEHVYSFPNK |
| 82 | EPHB4_583_595 | IGHGTKVYIDPFT |
| 83 | MK12_178_190 | ADSEMTGYVVTRW |
| 84 | VGFR1_1040_1052 | DFGLARDIYKNPD |
| 85 | PGFRB_572_584 | VSSDGHEYIYVDP |
| 86 | EPHA7_607_619 | TYIDPETYEDPNR |
| 87 | ERBB2_870_882 | LDIDETEYHADGG |
| 88 | LAT_194_206 | MESIDDYVNVPES |
| 89 | VINC_815_827 | KSFLDSGYRILGA |
| 90 | NCF1_313_325 | QRSRKRLSQDAYR |
| 91 | ERBB4_1277_1289 | IVAENPEYLSEFS |
| 92 | VGFR2_944_956 | RFRQGKDYVGAIP |
| 93 | NPT2A_501_513 | AKALGKRTAKYRW |
| 94 | C1R_199_211 | TEASGYISSLEYP |
| 95 | FABPH_13_25 | DSKNFDDYMKSLG |
| 96 | STAT1_694_706 | DGPKGTGYIKTEL |
| 97 | EPHA4_589_601 | LNQGVRTYVDPFT |
| 98 | VGFR1_1046_1058_Y1048F | DIFKNPDYVRKGD |
| 99 | INSR_992_1004 | YASSNPEYLSASD |
| 100 | NTRK2_509_521 | PVIENPQYFGITN |
| 101 | MBP_259_271 | FGYGGRASDYKSA |
| 102 | STA5A_687_699 | LAKAVDGYVKPQI |
| 103 | NTRK1_489_501 | HIIENPQYFSDAC |
| 104 | PTN11_539_551 | SKRKGHEYTNIKY |
| 105 | PLCG1_776_788 | EGRNPGFYVEANP |
| 106 | MK14_173_185 | RHTDDEMTGYVAT |
| 107 | MBP_263_275 | GRASDYKSAHKGF |
| 108 | RBL2_99_111 | VPTVSKGTVEGNY |
| 109 | RET_680_692 | AQAFPVSYSSSGA |
| 110 | EGFR_862_874 | LGAEEKEYHAEGG |
| 111 | INSR_1348_1360 | SLGFKRSYEEHIP |
| 112 | PRGR_545_557 | LRPDSEASQSPQY |
| 113 | SRC8_CHICK_470_482 | VSQREAEYEPETV |
| 114 | EPHA2_581_593 | QLKPLKTYVDPHT |
| 115 | VGFR1_1162_1174 | VQQDGKDYIPINA |
| 116 | MK01_198_210 | IMLNSKGYTKSID |
| 117 | STAT6_634_646 | MGKDGRGYVPATI |
| 118 | VGFR2_1207_1219_C1208S | VSDPKFHYDNTAG |
| 119 | ZBT16_621_633 | LRTHNGASPYQCT |
| 120 | EGFR_1118_1130 | APSRDPHYQDPHS |
| 121 | AMPE_5_17 | EREGSKRYCIQTK |
| 122 | FGFR3_641_653 | DVHNLDYYKKTTN |
| 123 | VGFR1_1206_1218 | GSSDDVRYVNAFK |
| 124 | EFS_246_258_Y253F | GGTDEGIFDVPLL |
| 125 | ODPAT_291_303 | SMSDPGVSYRTRE |
| 126 | STAT4_686_698 | TERGDKGYVPSVF |
| 127 | PLCG1_1246_1258 | EGSFESRYQQPFE |
| 128 | TNNT1_2_14 | SDTEEQEYEEEQP |
| 129 | EGFR_908_920 | MTFGSKPYDGIPA |
| 130 | EPHB1_921_933 | SAIKMVQYRDSFL |
| 131 | VGFR1_1235_1247 | ATSMFDDYQGDSS |
| 132 | STAT3_698_710 | DPGSAAPYLKTKF |
| 133 | CALM_93_105 | FDKDGNGYISAAE |
| 134 | DDR1_506_518 | LLLSNPAYRLLLA |
| 135 | EGFR_1062_1074 | EDSFLQRYSSDPT |
| 136 | EPHA4_921_933 | QAIKMDRYKDNFT |
| 137 | PERI_458_470 | QRSELDKSSAHSY |
| 138 | VGFR1_1049_1061 | KNPDYVRKGDTRL |

**Table 2: list of 48 peptide markers comprising phosphorylation sites used for determining the phosphatase activity, their sequence, extended sequence and SEQ ID NO. The name of the peptide markers refers to the associated proteins, the start and the end position of the amino acid sequence and the position of the nitrophosphate (shown in the extended sequence).**

| **SEQ ID NO** | **Name** | **Sequence** | **ExtendedSequence** |
|---|---|---|---|
| 139 | RET_1024_1033_Y1029npY | SDSLlYDDGL | SDSLI(NO2-pY)DDGL |
| 140 | RET_682_691_Y687npY | AFPVSYSSSG | AFPVS(NO2-pY)SSSG |
| 141 | FRK_382_391_Y387npY | DNEDIYESRH | DNEDI(NO2-pY)ESRH |
| 142 | MK01_182_191_Y187npY | FGLTEYVATR | FGLTE(NO2-pY)VATR |
| 143 | MK01_198_207_Y203npY | LNSKGYTKSI | LNSKG(NO2-pY)TKSI |
| 144 | CBL_695_704_Y700npY | EEDTEYBTPS | EEDTE(NO2-pY)BTPS |
| 145 | P85A_602_611_Y607npY | NTEDQYSLVE | NTEDQ(NO2-pY)SLVE |
| 146 | FAK1_572_581_Y577npY | EDSTYYKASK | EDSTY(NO2-pY)KASK |
| 147 | FAK1_572_581_Y576npY | EDSTYYKASK | EDST(NO2-pY)YKASK |
| 148 | CRK_216_226_Y221npY | PEPGPYAQPSV | PEPGP(NO2- pY)AQPSV |
| 149 | MET_1229_1239_Y1235npY | BYDKEYYSVHN | BYDKEY(NO2- pY)SVHN |
| 150 | MET_1229_1239_Y1234npY | BYDKEYYSVHN | BYDKE(NO2- pY)YSVHN |
| 151 | PGFRB_1016_1025_Y1021npY | EGDNDYIIPL | EGDND(NO2-pY)IIPL |
| 152 | MK12_180_189_Y185npY | SEBTGYVVTR | SEBTG(NO2-pY)VVTR |
| 153 | MBP_198_207_Y203npY | ARTAHYGSLP | ARTAH(NO2-pY)GSLP |
| 154 | MBP_263_272_Y268npY | GRASDYKSAH | GRASD(NO2-pY)KSAH |
| 155 | CADH2_780_789_Y785npY | EEDQDYDLSQ | EEDQD(NO2-pY)DLSQ |
| 156 | PDPK1_4_13_Y9npY | TTSQLYDAVP | TTSQL(NO2-pY)DAVP |
| 157 | ZAP70_287_296_Y292npY | LNSDGYTPEP | LNSDG(NO2-pY)TPEP |
| 158 | PGFRB_746_755_Y751npY | DESVDYVPBL | DESVD(NO2-pY)VPBL |
| 159 | GHR_591_600_Y595npY | PVPDYTSIHI | PVPD(NO2-pY)TSIHI |
| 160 | CSK_179_188_Y184npY | AQDEFYRSGW | AQDEF(NO2- pY)RSGW |
| 161 | SIGLEC2_817_826_Y822npY | DEGIHYSELI | DEGIH(NO2-pY)SELI |
| 162 | STAT3_701_709_Y705npY | SAAPYLKTK | SAAP(NO2-pY)LKTK |
| 163 | JAK2_1002_1011_Y1007npY | PQDKEYYKVK | PQDKE(NO2-pY)YKVK |
| 164 | LCK_389_399_Y394npY | IEDNEYTAREG | IEDNE(NO2- pY)TAREG |
| 165 | INSR_994_1004_Y999npY | SSNPEYLSASD | SSNPE(NO2- pY)LSASD |
| 166 | INSR_1350_1360_Y1355npY | GFKRSYEEHIP | GFKRS(NO2- pY)EEHIP |
| 167 | EGFR_1192_1202_Y1197npY | AENAEYLRVAP | AENAE(NO2- pY)LRVAP |
| 168 | EGFR_1011_1021_Y1016npY | VDADEYLIPQQ | VDADE(NO2- pY)LIPQQ |
| 169 | VGFR1_1208_1218_1213npY | SDDVRYVNAFK | SDDVR(NO2- pY)VNAFK |
| 170 | STA5A_689_699_Y694npY | KAVDGYVKPQI | KAVDG(NO2- pY)VKPQI |
| 171 | EGFR_1064_1074_Y1069npY | SFLQRYSSDPT | SFLQR(NO2- pY)SSDPT |
| 172 | ALK_1073_1083_Y1078npY | LQSPEYKLSKL | LQSPE(NO2- pY)KLSKL |
| 173 | TEC_514_524_Y519npY | FLDDQYTSSSG | FLDDQ(NO2- pY)TSSSG |
| 174 | CDK2_10_20_Y15npY | IGEGTYGVVYK | IGEGT(NO2- pY)GVVYK |
| 175 | RAF1_335_345_Y340npY | QRDSSYYWEIE | QRDSS(NO2- pY)YWEIE |
| 176 | PECA1_708_718_Y713npY | DTETVYSEVRK | DTETV(NO2- pY)SEVRK |
| 177 | STAT1_696_706_Y701npY | PKGTGYIKTEL | PKGTG(NO2-pY)IKTEL |
| 178 | ERBB2_1243_1253_Y1248npY | AENPEYLGLDV | AENPE(NO2- pY)LGLDV |
| 179 | PAXI_113_123_Y118npY | EEEHVYSFPNK | EEEHV(NO2- pY)SFPNK |
| 180 | EPOR_421_430_Y426npY | AASFEYTILD | AASFE(NO2-pY)TILD |
| 181 | CTNNB1_81_91_Y86npY | DIDGQYABTRA | DIDGQ(NO2- pY)ABTRA |
| 182 | ART_003_Y4pY | EAIYAAPFAKKK | EAI(pY)AAPFAKKK |
| 183 | TIE1_1002_1012_Y1007npY | RGEEVYVKKTB | RGEEV(NO2- pY)VKKTB |
| 184 | SELE_598_608_Y603npY | ESDGSYQKPSY | ESDGS(NO2- pY)QKPSY |
| 185 | BCR_172_182_Y177npY | AEKPFYVNVEF | AEKPF(NO2- pY)VNVEF |
| 186 | STAT3_701_709_Y705nY | SAAPYLKTK | SAAP(NO2-Y)LKTK |

**Table 3: list of 3 peptide markers comprising phosphorylation sites used for determining the kinase activity, their sequence and SEQ ID NO. The name of the peptide markers refers to the associated proteins and to the start and the end position of the amino acid sequence.**

| **SEQ ID NO** | **Name** | **Sequence** |
|---|---|---|
| 187 | ENOG_37_49 | SGASTGIYEALEL |
| 188 | SRC8_CHICK_476_488 | EYEPETVYEVAGA |
| 189 | SRC8_CHICK_492_504 | YQAEENTYDEYEN |

It should further be noted that according to a preferred embodiment of the present invention the peptide markers as listed in Table 1, Table 3 and/or Table 2 can be used as such for carrying out the methods according to the present invention. The present invention however also includes the use of analogs and combinations of these peptide markers for use in the method according to the present invention. The peptide marker analogs include peptide markers which show a sequence identity of more than 70%, preferably more than 80% and more preferably more than 90%.

In yet another embodiment, the present invention relates to a method for predicting the response of a patient diagnosed with melanoma cancer, to an immunotherapeutic antibody directed against CTLA-4, comprising the steps of:
(a) measuring the phosphatase activity or the phosphatase and kinase activity of a sample, obtained from said patient diagnosed with melanoma, by contacting said sample with at least one protein phosphatase substrate or at least one protein phosphatase and at least one protein kinase substrate, thereby providing a phosphorylation profile of said sample, said phosphorylation profile comprising the phosphorylation levels of phosphorylation sites present in at least the 10 peptide markers as defined by SEQ ID NOs: 139 - 148, and preferably in at least 10 peptide markers as listed in Table 1 and/or Table 3; and,
(c) calculating a classifier parameter from said phosphorylation profile; and,
(d) determining the response of said patient to said immunotherapeutic antibody directed against CTLA-4 on the basis of said classifier parameter.

By establishing a classifier parameter for determining the prediction of pharmacotherapy response of the melanoma patient the method of the present invention provides a criterion for analysing the results obtained from the method of the present invention. This criterion enables a person to provide a prediction or prognosis on the basis of a single or limited number of data. The person providing the prediction or prognosis does not have to interpret an entire set of data, but rather bases his conclusion on the basis of a single or limited number of criteria.

The term "classifier parameter" as used herein is a discriminating value which has been determined by establishing the phosphorylation profile of a sample obtained from a patient suffering from melanoma skin cancer. Said discriminating value identifies the prediction of response to pharmacotherapy of melanoma patients. The classifier parameter includes information regarding the phosphorylation level of several protein kinase and/or protein phosphatase substrates. Classification is a procedure in which individual items are placed into groups based on quantitative information on one or more characteristics inherent in the items (e.g. phosphorylation levels or profiles of a sample) and based on a training set of previously labelled items (clinical response to a pharmacotherapy). The classifier parameter is calculated by applying a "classifier" to the measured phosphorylation levels of a sample. Based on the classifying parameter a sample is assigned to (or predicted to belong to) a class (predicting the pharmacotherapy response of said patient). The classifier has been previously determined by comparing samples which are known to belong to the respective relevant classes. For instance the classifier may be a mathematical function that uses information regarding the phosphorylation level of several protein kinase and/or phosphatase substrates which individual protein kinase and/or protein phosphatase substrates can be statistically weighted based on the measured phosphorylation level of a number of protein kinase and/or protein phosphatase substrates (or values derived from that). Several methods are known in the art for developing a classifier including the neural network (Multi-layer Perceptron), support vector machines, k-nearest neighbours, Gaussian mixture model, naive bayes, decision tree, RBF classifiers, random forest, disciminant analysis, linear discriminant analysis, quadratic discriminant analysis, discriminant analysis - principal component analysis, partial least squares discriminant analysis, generalized distance regression and elastic net classification. The classifier parameter determined in this manner is valid for the same experimental setup in future individual tests.

It is not relevant to give an exact threshold value for the classifier parameter. A relevant threshold value can be obtained by correlating the sensitivity and specificity and the sensitivity/specificity for any threshold value. A threshold value resulting in a high sensitivity results in a lower specificity and vice versa. If one wants to increase the positive predictive value of the test to determine whether melanoma patient will respond to an immunotherapeutic antibody directed against CTLA-4, then the threshold value of the test can be changed which as a consequence will decrease the negative predictive value of the test to determine whether melanoma patient will not respond to an immunotherapeutic antibody directed against CTLA-4. If one wants to increase the negative predictive value of the test to determine whether melanoma patient will not respond to an immunotherapeutic antibody directed against CTLA-4, then the threshold value can be changed in the opposite direction which as a consequence will decrease the positive predictive value of the test to determine whether melanoma patient will respond to an immunotherapeutic antibody directed against CTLA-4

It is thus up to the diagnostic engineers to determine which level of positive predictive value/negative predictive value/sensitivity/specificity is desirable and how much loss in positive or negative predictive value is tolerable. The chosen threshold level could be dependent on other diagnostic parameters used in combination with the present method by the diagnostic engineers.

In yet another embodiment, the present invention relates to a method according to the present invention wherein said classifier parameter predicts the response of said patient to said immunotherapeutic antibody directed against CTLA-4 if said classifier parameter is above a first predetermined threshold level, and wherein said classifier parameter indicates non-response to said immunotherapeutic antibody directed against CTLA-4 of said patient if said classifier parameter is below a second predetermined threshold level.

According to another embodiment, the present invention relates to the method of the present invention wherein said differential phosphorylation level or said classifier parameter indicates a response, no-response or undetermined or intermediate prediction of said immunotherapeutic antibody directed against CTLA-4 or the effect of the an immunotherapeutic antibody directed against CTLA-4 of said patient.

As used in the present application the prediction of response to an immunotherapeutic antibody directed against CTLA-4 of melanoma patients is generally divided into two types of non-responders and responders and additionally some undetermined or intermediate responders. Whereas responders to a an immunotherapeutic antibody directed against CTLA-4 will survive longer or have additional clinical benefits (e.g. improved quality of life, prolonged progression free survival, etc.) due to the treatment, the non-responders to a an immunotherapeutic antibody directed against CTLA-4 will not benefit from the an immunotherapeutic antibody directed against CTLA-4. The method of the present invention specifically enables the distinction between responders and non-responders to a an immunotherapeutic antibody directed against CTLA-4.

The medicament as used in the method of the present invention is an immunotherapeutic antibody directed against CTLA-4.

As used herein, the term "immunotherapeutic antibody" refers to a type of antibody, preferably a monoclonal antibody, which binds to a specific cell or protein, preferably a cell surface protein, and thereby stimulates the immune system to attack those cells. The immunotherapeutic antibody is used in the treatment, cure, prevention, or diagnosis of disease or used to otherwise enhance physical or mental well-being.

As used herein, the term "immune checkpoint" refers to an inhibitory pathways hardwired into the immune system that is crucial for maintaining self-tolerance and modulating the duration and amplitude of physiological immune responses in peripheral tissues in order to minimize collateral tissue damage. Tumors can designate one or multiple immune-checkpoint pathways as a major mechanism of immune resistance, particularly against T cells that are specific for tumor antigens. Immune checkpoints can be blocked by antibodies. Examples of such immune checkpoints are CTLA-4, PD-1 and PD-L1.

More preferably the present invention relates to a method according to the present invention wherein said medicament is Ipilimumab,

Another a further embodiment, the kinase and/or phosphatase substrates carrying phosphorylation sites according to the present invention are located or immobilized on a solid support, and preferably a porous solid support. Preferably said immobilized kinase and/or phosphatase substrates carrying phosphorylation sites will be immobilized proteins, peptides or peptide mimetics. More preferably, the peptides are immobilized on a solid support.

As used herein "peptide" refers to a short truncated protein generally consisting of 2 to 100, preferably 2 to 30, more preferably 5 to 30 and even more preferably 13 to 18 naturally occurring or synthetic amino acids which can also be further modified including covalently linking the peptide bonds of the alpha carboxyl group of a first amino acid and the alpha amino group of a second amino acid by eliminating a molecule of water. The amino acids can be either those naturally occurring amino acids or chemically synthesized variants of such amino acids or modified forms of these amino acids which can be altered from their basic chemical structure by addition of other chemical groups which can be found to be covalently attached to them in naturally occurring compounds.

As used herein "protein" refers to a polypeptide made of amino acids arranged in a linear chain and joined together by peptide bonds between the carboxyl and amino groups of adjacent amino acid residues.

As used herein "peptide mimetics" refers to organic compounds which are structurally similar to peptides and similar to the peptide sequences list in Table 1, Table 3 and/or Table 2. The peptide mimetics are typically designed from existing peptides to alter the molecules characteristics. Improved characteristics can involve, for example improved stability such as resistance to enzymatic degradation, or enhanced biological activity, improved affinity by restricted preferred conformations and ease of synthesis. Structural modifications in the peptidomimetic in comparison to a peptide, can involve backbone modifications as well as side chain modification.

For measuring the kinase and/or phosphatase activity of the sample a large variety of methods and formats are known in the art. The kinase and/or phosphatase activity can for example be measured using ELISA and multiplex ELISA techniques, blotting methods, mass spectrometry, surface plasmon resonance, capillary electrophoresis, bead arrays, macroarrays, microarrays or any other method known in the art. Depending on the type of kinase and/or phosphatase activity measurement method the solid support on which the proteins, peptides or peptide mimetics are fixed may vary. Whereas in ELISA the protein kinase and/or protein phosphatase substrates are attached to the surface of the microtiterplates, in microarrays the protein kinase and/or protein phosphatase substrates are immobilized on and/or in the microarray substrate. Alternatively the substrates are synthesized in-situ direct on the microarray substrate.

In a preferred embodiment of the present invention the protein kinase and/or protein phosphatase substrates are immobilized on an array, and preferably a microarray of protein kinase and/or protein phosphatase substrates wherein the protein kinase and/or protein phosphatase substrates are immobilized onto a solid support or another carrier. The immobilization can be either the attachment or adherence of two or more protein kinase and/or protein phosphatase substrate molecules to the surface of the carrier including attachment or adherence to the inner surface of said carrier in the case of e.g. a porous or flow-through solid support.

In a preferred embodiment of the present invention, the array of protein kinase and/or protein phosphatase substrates is a flow-through array. The flow-through array as used herein could be made of any carrier material having oriented through-going channels as are generally known in the art, such as for example described in PCT patent publication WO 01/19517. Typically the carrier is made from a metal oxide, glass, silicon oxide or cellulose. In a particular embodiment the carrier material is made of a metal oxide selected from the group consisting of zinc oxide, zirconium oxide, tin oxide, aluminium oxide, titanium oxide and thallium; in a more particular embodiment the metal oxide consists of aluminium oxide.

Accordingly, in a further embodiment of the present invention said array is a Pamchip^{®}.

In a further embodiment, the present invention relates to a method according to the present invention wherein said solid support (microarray) comprises at least the 10 peptide markers as defined by SEQ ID NOs: 139 - 148, and/or at least 10 peptide markers as listed in Table 1 and/or Table 3 immobilized thereto.

In a further embodiment, the present invention relates to a method according to the present invention wherein said solid support (microarray) comprises each of the peptide as listed in Table 1 and Table 3, and Table 2 immobilized thereto.

In a further embodiment, the present invention relates to a method according to the present invention wherein said melanoma is an irresectable stage Illc or IV melanoma.

Phosphorylation levels can also be measured according to the invention, without the necessity to generate phosphorylation profiles thereof. Also for this embodiment, the amount and the type of peptides, proteins or peptide mimetics to be used is as described above.

The present invention also relates in another embodiment to a computer program product for use in conjunction with a computer having a processor and a memory connected to the processor, said computer program product comprising a computer readable storage medium having a computer program mechanism encoded thereon, wherein said computer program mechanism may be loaded into the memory of said computer and cause said computer to carry out the method according to the present invention.

The present invention further relates to a computer system comprising a processor, and a memory coupled to said processor and encoding one or more programs, wherein said one or more programs instruct the processor to carry out the methods according to the present invention.

The present invention also relates in another embodiment to a kit for determining the response of a patient diagnosed with melanoma, to an immunotherapeutic antibody directed against CTLA-4, comprising at least one array comprising at least 10 peptide markers as defined by SEQ ID NOs: 139 - 148, and preferably at least 10 peptide markers as listed in Table 1 and/or Table 3, and a computer readable storage medium having recorded thereon one or more programs for carrying out the method according to the present invention. In particular embodiment, the present invention relates to a kit for determining the response of a patient diagnosed with melanoma, to an immunotherapeutic antibody directed against CTLA-4, comprising two arrays, a first array comprising at least 10 peptide markers as listed in Table 1 and/or Table 3, and a second array comprising at least 10 peptide markers as defined by SEQ ID NOs: 139 - 148, and a computer readable storage medium having recorded thereon one or more programs for carrying out the method according to the present invention.

The present invention is hereafter exemplified by the illustration of particular, non-limiting examples.

### EXAMPLES

### EXAMPLE 1 - Example showing how responders and non-responders to targeted pharmacotherapy can be differentiated according to a phosphorylation inhibition profile.

A clinical study was conducted using blood samples from ten melanoma patients with a histologically or cytologically proven irresectable stage Illc or IV melanoma, which were treated with Ipilimumab. This allowed a comparison with the clinical responses.

In all the analyses, the first step was the isolation of PMBCs from the blood sample by Ficoll-Isopaque density centrifugation. Next, PBMCs were frozen in liquid N2 and subsequently lysed to allow extraction of the proteins. The kinase and phosphatase activity profiling was performed on a PamStation96 or a PamStation12 instrument that runs 96 (or 12) peptide microarrays. Each condition was run in multiple replicates. A range of 0.5 to 5 µg of total protein was tested in the kinase and phosphatase activity assays. 1 to 2 µg of total protein per assay was shown to be sufficient to obtain high signals. Besides the basal activity profiles, the same blood samples were analysed in the presence and absence of an protease inhibitor (Halt^{™} protease inhibitor cocktail which consists of an optimized concentration of six broad-spectrum protease inhibitors AEBSF, aprotinin, bestatin, E-64, leupeptin and pepstatin A (Thermo Scientific)) Addition of a protein kinase inhibitor to the protein tyrosine kinase assay and on the other hand, addition of a protein phosphatase inhibitor to the protein tyrosine phosphatase assay both showed a reduced signal (white no inhibition by X, black 100% inhibition by X) (Figure 1).

Figure 2 shows the residual phosphatase activity on the peptides markers as listed in Table 2 and the correlation of the phosphatase activity profile to the treatment outcome of the patients to Ipilimumab. The tumor samples of the five melanoma patients who clinically responded to the Ipilimumab medicament treatment are indicated as "R" (LAM-18, -22, - 23, -32 and -36) and the tumor samples of the five melanoma patients who clinically did not response to Ipilimumab medicament treatment are indicated as "NR" (LAM-05, -14, - 26, -29 and -35). High phosphatase activities were found in responders, low in poor responders. The analysis was performed completely blinded.

Figure 3 shows the residual kinase activity on the peptides markers as listed in Table 1 and the correlation of the kinase activity profile to the treatment outcome of the patients to Ipilimumab. The tumor samples of the five melanoma patients who clinically responded to the Ipilimumab medicament treatment are indicated as "R" (LAM-18, -22, -23, -32 and -36) and the tumor samples of the five melanoma patients who clinically did not response to Ipilimumab medicament treatment are indicated as "NR" (LAM-05, -14, -26, -29 and -35). High kinase activities were found in responders, low in poor responders. The analysis was performed completely blinded.

Interestingly, both the phosphatase and kinase activity profile provided a good prediction of the treatment outcome of patients treated with Ipilimumab. Using the method according to the present invention it was possible to divide patients which are either responsive or non-responsive to a treatment with Ipilimumab. In particular SEQ ID NO 1 up to SEQ ID NO 10 as listed in Table 1 and SEQ ID NO 139 up to SEQ ID NO 148 as listed in Table 2 were identified as key biomarkers for the differentiation between responsive and non-responsive patients.

### EXAMPLE 2 - Example showing how responders and non-responders to targeted pharmacotherapy can be differentiated according to kinase inhibition profiles.

A clinical study was conducted using PBMC (Peripheral Blood Mononuclear Cell) samples from melanoma patients treated with Pembrolizumab. The institute that provided the samples made an assessment of the clinical responses of the patients to the treatment. The patients with progressive disease (PD) were classified as Non-Responders (NR), those with stable disease (SD), partial response (PR) or complete response (CR) as Responders (R).

When peripheral whole blood is drawn for human immune system studies, it is often processed to remove red blood cells by density gradient centrifugation. Most commonly this method uses Ficoll Paque, a solution of high molecular weight sucrose polymers, a product of GE Healthcare Ltd. Ficoll separates whole blood into two fractions with densities above and below 1.077 g/ml. Peripheral blood mononuclear cells (PBMC) are the populations of cells that remain at the less dense, upper interface of the Ficoll layer, often referred to as the buffy coat and are the cells collected when the Ficoll fractionation method is used. PBMC consist of: 70 - 90% lymphocytes (T cells, B cells, and NK cells), 10 - 30% monocytes and 1 - 2% dendritic cells.

The samples were obtained by drawing blood in heparine tubes. After Ficoll fractionation PBMCs were frozen in 10% DMSO / 90% FBS according to standard procedures. The LDH value and composition of the blood was determined before or at the start of the therapy. All samples contained approximately ~1 ×10⁷ PBMCs/vial.

After lysis and protein determination the PTP (Protein Tyrosine Phosphatase) and PTK (Protein Tyrosine Kinase) activity was determined for all samples using 2 ug of protein per array. Additionally the PTP assay was performed with 0.5 ug protein/array. The kinase and phosphatase activity profiling was performed on a PamStation96 or a PamStation12 instrument that runs 96 (or 12) peptide microarrays. Each condition was run in multiple replicates. Analysis was performed with basal PTP and PTK signals and VSN normalized signals.

Samples with low PTK activity (two 1st line Pembrolizumab responders) were excluded from the analysis. T-tests were performed to distinguish responders from non- responders on the basal signals and on the signals after VSN normalisation. Analysis of the normalised values for PTK showed differences between 1st line Pembrolizumab responders and non-responders. 7 peptides had lower signals in responders and 16 peptides had higher signals in responders (p<0.05).

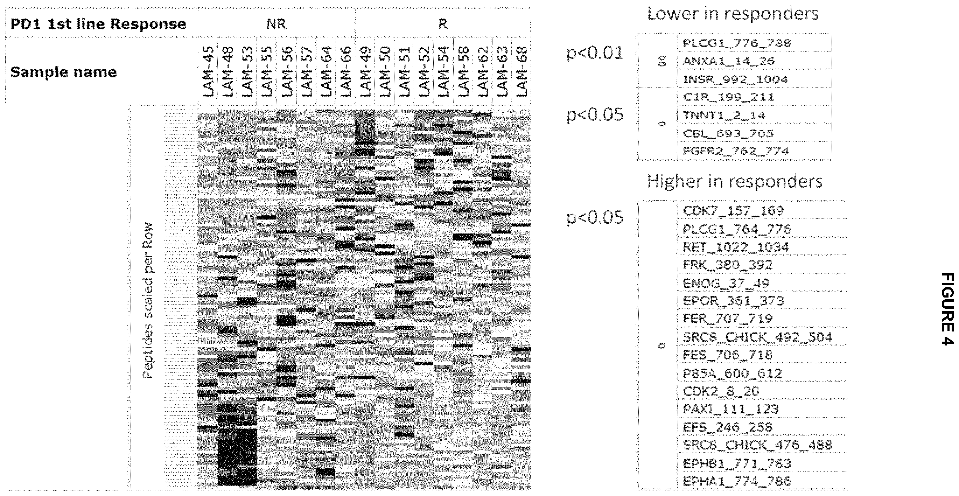

Figure 4 shows a heatmap of z-score normalized peptides for 1st line Pembrolizumab responders vs non-responders. Peptides with significantly different (p<0.05) signals are shown on the right hand side. In particular SEQ ID NO 105, 17, 99, 94, 128, 24, 11, 1, 72, 16, 69, 187, 45, 22, 189, 48, 80, 55, 81, 124, 188, 49 and 60 as listed in Table 1 and Table 3, were identified as key biomarkers for the differentiation between responsive and non-responsive patients.

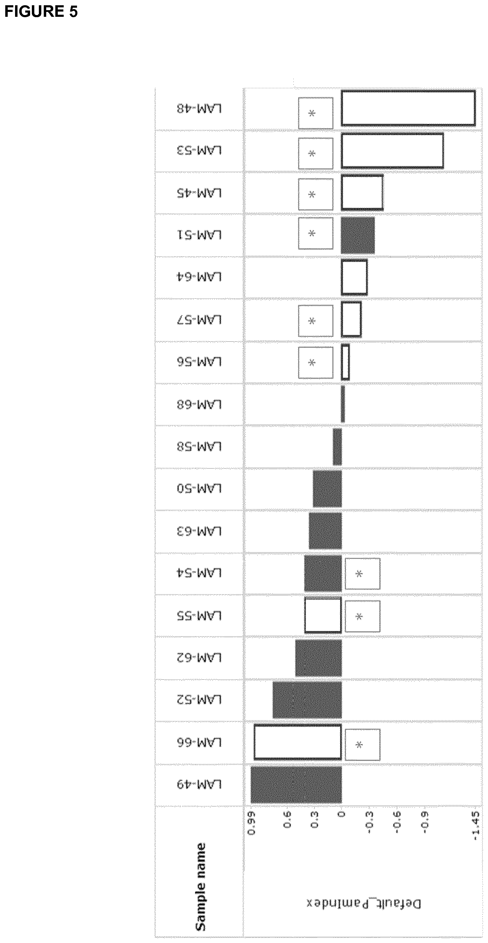

Partial Least Square Discriminant Analysis (PLS-DA) was used to make a model for response prediction. The response for each sample was predicted with Leave One Out Cross Validation (LOOCV). The results of this analysis are shown in Figure 5 (Grey - Responders; White - Non-responders; * Metastasis present). For 13 out of 17 samples the prediction was correct (76%). The probability of obtaining such a result by chance is p<0.02. It should be noted that most non-responders to Pembrolizumab had brain metastases. LAM-54 also had a brain metastasis, but that was removed before treatment started. LAM-51, who was predicted incorrectly, stood out because the basophile count was 0.

Interestingly, the kinase activity profile provided a good prediction of the treatment outcome of patients treated with Pembrolizumab. Using the method according to the present invention it was possible to divide patients which are either responsive or non-responsive to a treatment with Pembrolizumab. In SEQ ID NO 105, 17, 99, 94, 128, 24, 11, 1, 72, 16, 69, 187, 45, 22, 189, 48, 80, 55, 81, 124, 188, 49 and 60 as listed in Table 1 and Table 3 were identified as key biomarkers for the differentiation between responsive and non-responsive patients.

## Claims

1. A method for predicting the response of a patient diagnosed with melanoma cancer, to an immunotherapeutic antibody directed against CTLA-4, comprising the steps of:
(a) measuring the phosphatase activity of a sample, obtained from said patient diagnosed with melanoma, by contacting said sample with at least one protein phosphatase substrate, thereby providing a phosphorylation profile of said sample, said phosphorylation profile comprising the phosphorylation levels of phosphorylation sites present in at least the peptide markers as defined by SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147 and SEQ ID NO: 148; wherein said sample is obtained from a blood sample of said patient diagnosed with melanoma; and,
(b) determining from said phosphorylation profile the response of said patient to said immunotherapeutic antibody directed against CTLA-4.

2. Method according to claims 1, wherein said blood sample comprises peripheral blood mononuclear cells.

3. Method according to claim 1 or 2, wherein said phosphorylation profiles comprise the phosphorylation levels of phosphorylation sites present in the peptide markers as defined by SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147 and SEQ ID NO: 148 and at least 20 further peptide markers as listed in Table 2.

4. Method according to any one of claims 1 to 3, wherein said phosphorylation profiles comprise the phosphorylation levels of phosphorylation sites present in all the peptide markers as listed in Table 2.

5. Method according to any one of claims 1 to 4, further comprising measuring the kinase activity of said sample, by contacting said sample with at least one protein kinase substrate and wherein said phosphorylation profile further comprises the phosphorylation levels of phosphorylation sites present in at least 10 peptide markers as listed in Table 1 and/or Table 3.

6. Method according to any one of claims 1 to 5, wherein step (b) is replaced by a step (c) calculating a classifier parameter from said phosphorylation profile; and a step (d) determining the response of said patient to said immunotherapeutic antibody directed against CTLA-4 on the basis of said classifier parameter.

7. Method according to claim 6, wherein said classifier parameter indicates said patient being a good responder to said immunotherapeutic antibody directed against CTLA-4if said classifier parameter is above a first predetermined threshold level, and wherein said classifier parameter indicates said patient being a poor responder to said immunotherapeutic antibody directed against CTLA-4 if said classifier parameter is below a second predetermined threshold level.

8. Method according to claims 6 or 7, wherein step (b) is replaced by a step (e) comparing said phosphorylation profile to a first and a second reference phosphorylation profile; said first reference phosphorylation profile being representative for a good responder to said immunotherapeutic antibody directed against CTLA-4 and said second reference phosphorylation profile being representative for a poor responder to said immunotherapeutic antibody directed against CTLA-4; and a step (f) determining response of said patient to said immunotherapeutic antibody directed against CTLA-4on the basis of the comparison of said phosphorylation profile with said first and said second reference phosphorylation profile.

9. The method according to any of claims 6 to 8, wherein said phosphorylation profile or said classifier parameter indicates good response, poor response or undetermined response of said patient to said immunotherapeutic antibody directed against CTLA-4.

10. The method according to any of claims 1 to 9, wherein said melanoma is an irresectable stage IIIc or IV melanoma.

11. The method according to any of claims 1 to 10, wherein from the measurements in step (a) the toxicity of said immunotherapeutic antibody directed against CTLA-4 in said patient is determined.

12. The method according to any one of claim 1 to 11, wherein the immunotherapeutic antibody directed against CTLA-4 is Ipililumab.

13. A computer program product for use in conjunction with a computer having a processor and a memory connected to the processor, said computer program product comprising a computer readable storage medium having a computer program mechanism encoded thereon, wherein said computer program mechanism may be loaded into the memory of said computer and cause said computer to carry out the method of any one of claims 1 to 12.

## Patentansprüche

1. Verfahren zur Vorhersage des Ansprechens eines mit Melanomkrebs diagnostizierten Patienten auf einen immuntherapeutischen Antikörper, der gegen CTLA-4 gerichtet ist, umfassend die Schritte:
(a) Messen der Phosphatase-Aktivität einer Probe, die von dem mit Melanom diagnostizierten Patienten erhalten wurde, indem die Probe mit wenigstens einem Proteinphosphatase-Substrat in Kontakt gebracht wird, wodurch ein Phosphorylierungsprofil der Probe bereitgestellt wird, wobei das Phosphorylierungsprofil die Phosphorylierungsniveaus von Phosphorylierungsstellen umfasst, die in wenigstens den Peptidmarkern wie definiert durch SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147 und SEQ ID NO: 148 vorhanden sind; wobei die Probe von einer Blutprobe des mit Melanom diagnostizierten Patienten erhalten wird; und
(b) Bestimmen des Ansprechens des Patienten auf den gegen CTLA-4 gerichteten immuntherapeutischen Antikörper anhand des Phosphorylierungsprofils.

2. Verfahren nach Anspruch 1, wobei die Blutprobe mononukleäre Zellen des peripheren Bluts umfasst.

3. Verfahren nach Anspruch 1 oder 2, wobei die Phosphorylierungsprofile die Phosphorylierungsniveaus von Phosphorylierungsstellen umfassen, die in den Peptidmarkern wie definiert durch SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147 und SEQ ID NO: 148 und wenigstens 20 weiteren, wie in Tabelle 2 angegebenen Peptidmarkern vorhanden sind.

4. Verfahren nach einem der Ansprüche 1 bis 3, wobei die Phosphorylierungsprofile die Phosphorylierungsniveaus von Phosphorylierungsstellen umfassen, die in allen wie in Tabelle 2 angegebenen Peptidmarkern vorhanden sind.

5. Verfahren nach einem der Ansprüche 1 bis 4, ferner umfassend Messen der Kinase-Aktivität der Probe, indem die Probe mit wenigstens einem Proteinkinase-Substrat in Kontakt gebracht wird und wobei das Phosphorylierungsprofil ferner die Phosphorylierungsniveaus von Phosphorylierungsstellen umfasst, die in wenigstens 10 wie in Tabelle 1 und/oder Tabelle 3 angegebenen Peptidmarkern vorhanden sind.

6. Verfahren nach einem der Ansprüche 1 bis 5, wobei Schritt (b) durch einen Schritt (c), bei dem ein Klassifikatorparameter anhand des Phosphorylierungsprofils berechnet wird, und einen Schritt (d), bei dem das Ansprechen des Patienten auf den gegen CTLA-4 gerichteten immuntherapeutischen Antikörper auf der Basis des Klassifikatorparameters bestimmt wird, ersetzt ist.

7. Verfahren nach Anspruch 6, wobei durch den Klassifikatorparameter ein gutes Ansprechen des Patienten auf den gegen CTLA-4 gerichteten immuntherapeutischen Antikörper angezeigt wird, falls der Klassifikatorparameter oberhalb eines ersten vorbestimmten Schwellenniveaus liegt, und wobei durch den Klassifikatorparameter ein schlechtes Ansprechen des Patienten auf den gegen CTLA-4 gerichteten immuntherapeutischen Antikörper angezeigt wird, falls der Klassifikatorparameter unterhalb eines zweiten vorbestimmten Schwellenniveaus liegt.

8. Verfahren nach Anspruch 6 oder 7, wobei Schritt (b) durch einen Schritt (e), bei dem das Phosphorylierungsprofil mit einem ersten und einem zweiten Referenzphosphorylierungsprofil verglichen wird, wobei das erste Referenzphosphorylierungsprofil für ein gutes Ansprechen auf den gegen CTLA-4 gerichteten immuntherapeutischen Antikörper und das zweite Referenzphosphorylierungsprofil für ein schlechtes Ansprechen auf den gegen CTLA-4 gerichteten immuntherapeutischen Antikörper repräsentativ ist, und einen Schritt (f), bei dem das Ansprechen des Patienten auf den gegen CTLA-4 gerichteten immuntherapeutischen Antikörper auf der Grundlage des Vergleichs des Phosphorylierungsprofils mit dem ersten und dem zweiten Referenzphosphorylierungsprofil bestimmt wird, ersetzt ist.

9. Verfahren nach einem der Ansprüche 6 bis 8, wobei das Phosphorylierungsprofil oder der Klassifikatorparameter ein gutes Ansprechen, ein schlechtes Ansprechen oder ein unbestimmtes Ansprechen des Patienten auf den gegen CTLA-4 gerichteten immuntherapeutischen Antikörper anzeigt.

10. Verfahren nach einem der Ansprüche 1 bis 9, wobei es sich bei dem Melanom um ein inoperables Melanom im Stadium IIIc oder IV handelt.

11. Verfahren nach einem der Ansprüche 1 bis 10, wobei anhand der Messungen in Schritt (a) die Toxizität des gegen CTLA-4 gerichteten immuntherapeutischen Antikörpers beim Patienten bestimmt wird.

12. Verfahren nach einem der Ansprüche 1 bis 11, wobei es sich bei dem gegen CTLA-4 gerichteten immuntherapeutischen Antikörper um Ipililumab handelt.

13. Computerprogrammprodukt zur Verwendung in Verbindung mit einem Computer mit einem Prozessor und einem mit dem Prozessor verbundenen Speicher, wobei das Computerprogrammprodukt ein computerlesbares Speichermedium mit einem darauf codierten Computerprogrammmechanismus umfasst, wobei der Computerprogrammmechanismus in den Speicher des Computers geladen werden kann und den Computer veranlassen kann, das Verfahren gemäß einem der Ansprüche 1 bis 12 auszuführen.

## Revendications

1. Procédé pour prédire la réponse d'un patient diagnostiqué d'un cancer du mélanome, à un anticorps immunothérapeutique dirigé contre CTLA-4, comprenant les étapes de :
(a) mesure de l'activité de phosphatase d'un échantillon, obtenu dudit patient diagnostiqué d'un mélanome, par mise en contact dudit échantillon avec au moins un substrat de phosphatase protéique, fournissant ainsi un profil de phosphorylation dudit échantillon, ledit profil de phosphorylation comprenant les niveaux de phosphorylation des sites de phosphorylation présents dans au moins les marqueurs peptidiques tels que définis par les SEQ ID NO : 139, SEQ ID NO : 140, SEQ ID NO : 141, SEQ ID NO : 142, SEQ ID NO : 143, SEQ ID NO : 144, SEQ ID NO : 145, SEQ ID NO : 146, SEQ ID NO : 147 et SEQ ID NO : 148 ; dans lequel ledit échantillon est obtenu à partir d'un échantillon de sang dudit patient diagnostiqué d'un mélanome ; et,
(b) détermination à partir dudit profil de phosphorylation de la réponse dudit patient audit anticorps immunothérapeutique dirigé contre CTLA-4.

2. Procédé selon la revendication 1, dans lequel ledit échantillon de sang comprend des cellules mononucléaires de sang périphérique.

3. Procédé selon la revendication 1 ou 2, dans lequel lesdits profils de phosphorylation comprennent les niveaux de phosphorylation des sites de phosphorylation présents dans les marqueurs peptidiques tels que définis par les SEQ ID NO : 139, SEQ ID NO : 140, SEQ ID NO : 141, SEQ ID NO : 142, SEQ ID NO : 143, SEQ ID NO : 144, SEQ ID NO : 145, SEQ ID NO : 146, SEQ ID NO : 147 et SEQ ID NO : 148 et au moins 20 autres marqueurs peptidiques tels que listés dans le Tableau 2.

4. Procédé selon l'une quelconque des revendications 1 à 3, dans lequel lesdits profils de phosphorylation comprennent les niveaux de phosphorylation des sites de phosphorylation présents dans tous les marqueurs peptidiques tels que listés dans le Tableau 2.

5. Procédé selon l'une quelconque des revendications 1 à 4, comprenant en outre la mesure de l'activité de kinase dudit échantillon, par mise en contact dudit échantillon avec au moins un substrat de protéine kinase et dans lequel ledit profil de phosphorylation comprend en outre les niveaux de phosphorylation de sites de phosphorylation présents dans au moins 10 marqueurs peptidiques tels que listés dans le Tableau 1 et/ou le Tableau 3.

6. Procédé selon l'une quelconque des revendications 1 à 5, dans lequel l'étape (b) est remplacée par une étape (c) de calcul d'un paramètre classificateur à partir dudit profil de phosphorylation ; et une étape (d) de détermination de la réponse dudit patient audit anticorps immunothérapeutique dirigé contre CTLA-4 sur la base dudit paramètre classificateur.

7. Procédé selon la revendication 6, dans lequel ledit paramètre classificateur indique que ledit patient répond bien audit anticorps immunothérapeutique dirigé contre CTLA-4 si ledit paramètre classificateur est supérieur à un premier niveau seuil prédéterminé, et dans lequel ledit paramètre classificateur indique que ledit patient répond mal audit anticorps immunothérapeutique dirigé contre CTLA-4 si ledit paramètre classificateur est inférieur à un deuxième niveau seuil prédéterminé.

8. Procédé selon la revendication 6 ou 7, dans lequel l'étape (b) est remplacée par une étape (e) de comparaison dudit profil de phosphorylation à un premier et un deuxième profil de phosphorylation de référence ; ledit premier profil de phosphorylation de référence étant représentatif d'un bon répondant audit anticorps immunothérapeutique dirigé contre CTLA-4 et ledit deuxième profil de phosphorylation de référence étant représentatif d'un mauvais répondant audit anticorps immunothérapeutique dirigé contre CTLA-4 ; et une étape (f) de détermination de la réponse dudit patient audit anticorps immunothérapeutique dirigé contre CTLA-4 sur la base de la comparaison dudit profil de phosphorylation avec ledit premier et ledit deuxième profil de phosphorylation de référence.

9. Procédé selon l'une quelconque des revendications 6 à 8, dans lequel ledit profil de phosphorylation ou ledit paramètre classificateur indique une bonne réponse, une mauvaise réponse ou une réponse indéterminée dudit patient audit anticorps immunothérapeutique dirigé contre CTLA-4.

10. Procédé selon l'une quelconque des revendications 1 à 9, dans lequel ledit mélanome est un mélanome de stade IIIc ou IV non résécable.

11. Procédé selon l'une quelconque des revendications 1 à 10, dans lequel, à partir des mesures de l'étape (a), la toxicité dudit anticorps immunothérapeutique dirigé contre CTLA-4 chez ledit patient est déterminée.

12. Procédé selon l'une quelconque des revendications 1 à 11, dans lequel l'anticorps immunothérapeutique dirigé contre CTLA-4 est Ipililumab.

13. Produit programme informatique destiné à être utilisé conjointement avec un ordinateur ayant un processeur et une mémoire connectée au processeur, ledit produit programme informatique comprenant un support de stockage lisible par ordinateur ayant un mécanisme de programme informatique codé sur celui-ci, dans lequel ledit mécanisme de programme informatique peut être chargé dans la mémoire dudit ordinateur et amener ledit ordinateur à réaliser le procédé selon l'une quelconque des revendications 1 à 12.
